(19) Europäisches Patentamt
European Patent Office
Office européen des brevets

(11) **EP 1 549 937 B1**

(12) **EUROPÄISCHE PATENTSCHRIFT**

(45) Veröffentlichungstag und Bekanntmachung des Hinweises auf die Patenterteilung:
**20.03.2013 Patentblatt 2013/12**

(21) Anmeldenummer: **03787628.1**

(22) Anmeldetag: **03.07.2003**

(51) Int Cl.:
*G01N 29/02* (2006.01)    *G01N 33/543* (2006.01)
*G01N 29/036* (2006.01)

(86) Internationale Anmeldenummer:
**PCT/DE2003/002226**

(87) Internationale Veröffentlichungsnummer:
**WO 2004/017063 (26.02.2004 Gazette 2004/09)**

(54) **Vorrichtung und Verfahren zur Detektion einer Substanz mithilfe eines piezoelektrischen Dünnfilmresonators**

Device and method for detecting a substance with a piezoelectric thin film resonator

Dispositif et procédé pour detecter une substance avec un résonateur en film mince piézoélectrique

(84) Benannte Vertragsstaaten:
**DE FR GB**

(30) Priorität: **19.07.2002 DE 10232940**
**28.02.2003 DE 10308975**

(43) Veröffentlichungstag der Anmeldung:
**06.07.2005 Patentblatt 2005/27**

(73) Patentinhaber: **Siemens Aktiengesellschaft**
**80333 München (DE)**

(72) Erfinder:
• **GABL, Reinhard**
**8542 St. Peter in Sulmtal (AT)**
• **WERSING, Wolfram**
**83346 Bergen (DE)**
• **FEUCHT, Hans-Dieter**
**71272 Renningen (DE)**
• **ZEININGER, Heinrich**
**90587 Obermichelbach (DE)**

(56) Entgegenhaltungen:
**EP-A- 1 143 241    US-A- 5 075 641**
**US-A- 5 932 953**

• **R.M. WHITE: "Surface acoustic wave sensors" ULTRASONICS SYMPOSIUM 1985, 1985, Seiten 490-494, XP001155517**
• **FERRARI V ET AL: "Multisensor array of mass microbalances for chemical detection based on resonant piezo-layers of screen-printed PZT" SENSORS AND ACTUATORS B, ELSEVIER SEQUOIA S.A., LAUSANNE, CH, Bd. 68, Nr. 1-3, 25. August 2000 (2000-08-25), Seiten 81-87, XP004216596 ISSN: 0925-4005 in der Anmeldung erwähnt**
• **I. WILLNER, E. KATZ: "Redoxproteinschichten auf leitenden Trägern - Systeme für bioelektronische Anwendungen" ANGEW. CHEM., Bd. 112, 2000, Seiten 1230-1269, XP002269190 in der Anmeldung erwähnt**
• **LIANG HONGWEI ET AL: 'Material parameter characterization of multilayer LTCC and implementation of high Q resonators' IEEE MTT S INT MICROWAVE SYMP DIG; IEEE MTT-S INTERNATIONAL MICROWAVE SYMPOSIUM DIGEST 1999 IEEE, PISCATAWAY, NJ, USA Bd. 4, 1999, Seiten 1901 - 1904, XP010343609**

**Beschreibung**

Die Erfindung betrifft eine Vorrichtung zur Detektion mindestens einer Substanz eines Fluids, aufweisend mindestens einen piezoakustischen Resonator mit mindestens einer piezoelektrischen Schicht, einer an der piezoelektrischen Schicht angeordneten Elektrode, mindestens einer an der piezoelektrischen Schicht angeordneten weiteren Elektrode und einem Oberflächenabschnitt zur Sorption der Substanz des Fluids, wobei die piezoelektrische Schicht, die Elektroden und der Oberflächenabschnitt derart aneinander angeordnet sind, dass eine elektrische Ansteuerung der Elektroden zu einer Schwingung des Resonators mit einer Resonanzfrequenz führt und die Resonanzfrequenz abhängig ist von einer am Oberflächenabschnitt adsorbierten Menge der Substanz. Daneben wird ein Verfahren zur Detektion mindestens einer Substanz eines Fluids unter Verwendung der Vorrichtung angegeben.

In der modernen biologischen Analysetechnik und in der medizinischen Diagnostik werden in zunehmenden Maße Biosensoren eingesetzt. Ein Biosensor besteht aus einem biologischen Erkennungssystem für eine biologische Substanz und einem sogenannten physikalischen Transducer. Über das biologische Erkennungssystem erfolgt ein "Erkennen" der Substanz. Dieses "Erkennen" wird mit Hilfe des physikalischen Transducers in ein elektronisches Signal umgewandelt. Häufig eingesetzte biologische Erkennungssysteme sind Antikörper, Enzyme und Nukleinsäuren. Die biologischen Erkennungssysteme werden dabei meist in annähernd zweidimensionalen Schichten auf dem Transducer immobilisiert (fixiert). Ein Immobilisieren (Fixieren) kann dabei durch kovalente Bindungen, durch Affinitätswechselwirkungen und durch hydrophile oder hydrophobe Wechselwirkungen erfolgen. Einen Überblick über einen Aufbau annähernd zweidimensionaler biologischer Erkennungsschichten geben I. Willner und E. Katz in Angew. Chem. 112 (2000), 1230 bis 1269.

Eine Vorrichtung und ein Verfahren der eingangs genannten Art ist aus C. Kößlinger et al., Biosensors & Bioelectronics, 7 (1992), S. 397 bis 404 bekannt. Der Oberflächenabschnitt des Resonators stellt ein Erkennungssystem für eine Substanz dar. Der piezoelektrische Resonator fungiert als physikalischer Transducer. Die piezoelektrische Schicht des bekannten Resonators besteht aus einem Quarzkristall. An dem Quarzkristall sind Elektroden aus Gold angebracht. Durch eine elektrische Ansteuerung der Elektroden wird der Quarzkristall zu akustischen Volumenwellen (Bulk Akoustic Waves) in Form von Dickenscherschwingungen angeregt. Die Resonanzfrequenz beträgt etwa 20 MHz. Eine der Elektroden bildet den Oberflächenabschnitt zur Sorption der Substanz des Fluids. Die Substanz ist ein makromolekulares Protein, das sich in einer Flüssigkeit befindet und das an der Elektrode physikalisch adsorbiert wird. Durch die Adsorption des Proteins ändert sich die Masse und damit die Resonanzfrequenz des Resonators. Für die Änderung der Resonanzfrequenz ($\Delta f$) in Abhängigkeit von der Änderung der adsorbierten Menge der Substanz pro Flächeneinheit ($\Delta m$) gilt folgender allgemeine Zusammenhang (vergleiche G. Sauerbrey, Zeitschrift für Physik, 155 (1959), S. 206 - 222):

$$S = \frac{\Delta f}{\Delta m} = c\,\frac{f_0}{m} \propto f_0^2 \qquad\qquad (1)$$

Dabei ist S die Massensensitivität des Resonators, $f_0$ die Resonanzfrequenz des Resonators ohne adsorbierte Substanz, c ist eine materialspezifische Konstante und m die Masse des Resonators pro Flächeneinheit. Die Massensensitivität ist proportional zum Quadrat der Resonanzfrequenz des Resonators. Bei einer relativ niedrigen Resonanzfrequenz $f_0$ von etwa 20 MHz kann die Massensensitivität der bekannten Vorrichtung auf etwa 1 Hz·ng$^{-1}$·cm$^2$ abgeschätzt werden.

Eine Vorrichtung und ein Verfahren der genannten Art sind auch aus V. Ferrari et al., Sensors and Actuators, B 68 (2000), S. 81-87 bekannt. Die Vorrichtung fungiert als Massensensor zur Detektion einer chemischen Substanz. Die piezoelektrische Schicht ist eine Bleizirkonattitanat (PZT)-Schicht. An gegenüberliegenden Seiten der PZT-Schicht sind schichtförmige Elektroden (Elektrodenschichten) aus einer Silberpalladiumlegierung angebracht. Die Elektroden und die PZT-Schicht bilden den piezoakustischen Resonator. Durch eine elektrische Ansteuerung der Elektroden ist der Resonator zu einer Längsschwingung (longitudinale Schwingung) entlang der Schichtdicke der PZT-Schicht anregbar.

Der bekannte Resonator weist einen Oberflächenabschnitt auf, an dem eine Substanz sorbiert werden kann. Dazu verfügt der Resonator über einen den Oberflächenabschnitt bildende, chemisch sensitive Beschichtung. Die chemisch sensitive Beschichtung ist ein Polymerfilm, der auf einer der Elektroden aufgebracht ist. Der Polymerfilm ist beispielsweise Polystyrol oder Polymethylacrylat. Auf diesen Polymerfilmen können verschiedene Substanzen, beispielsweise Kohlenwasserstoffe, adsorbiert werden. Durch die Adsorption ändert sich die Masse des Resonators. Als Folge davon ändert sich die Resonanzfrequenz des Resonators. Ein Ausmaß der Änderung der Resonanzfrequenz hängt von der adsorbierten Menge der Substanz ab. Je mehr Substanz adsorbiert ist, desto größer ist die Änderung der Resonanzfrequenz.

Die Schichtdicke der PZT-Schicht des Resonators beträgt ungefähr 100 $\mu$m. Die Elektroden sind etwa 10 $\mu$m dick. Der Polymerfilm ist beispielsweise mit einer Dicke von etwa 3 $\mu$m aufgebracht. Eine laterale Ausdehnung des Resonators beträgt etwa 6 mm. Die Resonanzfrequenz des Resonators beträgt ungefähr 7 MHz. Die bekannte Vorrichtung mit dem piezoakustischen Resonator eignet sich zur Detektion einer Substanz eines Fluids. Das Fluid ist entweder eine Flüssigkeit oder ein Gas bzw. Gasgemisch.

Der Resonator der Vorrichtung ist auf einem Substrat aus Aluminiumoxid aufgebracht. Zum Herstellen des Resonators bzw. zum Aufbringen des Resonators auf dem Substrat wird auf die sogenannte Dickfilmtechnologie (Thick Film Technology, TFT) zurückgegriffen. Aufgrund einer mit der Dickfilmtechnologie erzielbaren Auflösung ist eine Miniaturisierung der Vorrichtung begrenzt. Die zunehmende Miniaturisierung ist aber wünschenswert. Beispielsweise werden eine Vielzahl von Resonatoren zu einer Resonatormatrix (Resonatorarray) zusammengefasst. Jeder der Resonatoren bildet ein Matrixelement der Resonatormatrix. Um möglichst viele Matrixelemente auf einem Substrat einer gegebenen Größe anordnen zu können, sind möglichst kleine Resonatorelemente nötig.

Neben der im Hinblick auf die Miniaturisierung ungünstigen Größe des Resonators zeichnet sich die bekannte Vorrichtung aufgrund der relativ niedrigen Resonanzfrequenz des Resonators durch eine relativ niedrige Massensensitivität aus (vgl. Gleichung 1). Eine niedrige Konzentration der Substanz des Fluids oder eine geringe Änderung der Konzentration der Substanz im Fluid kann nur mit einem relativ großen Fehler bestimmt werden.

Aus H. Baltes, Proceedings of the IEEE, Vol. 86, No. 8, August 1998, Seiten 1660-1678 ist ein sogenannter Flexural Plate Wave (FPW)-Sensor bekannt. Der Sensor ist eine Vorrichtung zur Detektion einer Substanz. Die Vorrichtung verfügt über einen piezoakustischen Resonator, der auf einem Halbleitersubstrat aus Silizium aufgebracht ist. Zum Herstellen der Vorrichtung werden Dampfabscheideverfahren, CMOS (Complementary Metal Oxyde Semiconductor)-Technologie und Front- bzw. Rückseitenätzen des Halbleitersubstrats (bulk micromachining) eingesetzt. Die Elektroden und die piezoelektrische Schicht sind auf dem Halbleitersubstrat in Form eines sogenannten Auslegers derart angeordnet, dass eine elektrische Ansteuerung der Elektroden zu einer Querschwingung des Resonators mit einer Resonanzfrequenz von etwa 140 kHz führt. Der Resonator verfügt über eine chemisch sensitive Beschichtung aus Polyurethan oder Polysiloxan. Diese Polymere sind für die Adsorption und damit den Nachweis von Kohlenwasserstoffen mit Halogenen geeignet. Das Fluid ist insbesondere gasförmig. Wenn das Fluid an dem durch eines der Polymere gebildeten Oberflächenabschnitt vorbeigeleitet wird, werden die Kohlenwasserstoffe an dem Oberflächenabschnitt adsorbiert. In Abhängigkeit von der Konzentration der Kohlenwasserstoffe ändert sich die Masse des Resonators und damit auch die Resonanzfrequenz des Resonators. Die laterale Ausdehnung des Resonators ist relativ klein. Sie beträgt beispielsweise 300 $\mu$m. Allerdings zeichnet sich der Resonator aufgrund der relativ niedrigen Resonanzfrequenz von etwa 140 kHz durch eine relativ niedrige Massensensitivität aus.

Aus der US-A-5 932 953 ist eine Vorrichtung zur Detektion einer Substanz eines Fluids mit einem BAW-Resonator in Dünnfilmbauweise bekannt. Der Resonator ist auf einem Halbleitersubstrat aufgebracht. Direkt unterhalb des Resonators verfügt das Halbleitersubstrat eine Ausnehmung. Diese Ausnehmung fungiert als Einrichtung zur akustischen Isolation des Resonators und des Substrats.

Aus der US-A-5 075 641 ist ein Hochfrequenz-Oszillator mit einem integrierten Dünnfilmresonator bekannt, wobei ein Halbleitersubstrat mit einer metallischen Durchkontaktierung vorgesehen ist, die gleichzeitig als eine Elektrode des Dünnfilmresonators dient.

Aufgabe der vorliegenden Erfindung ist es, eine Vorrichtung zur Detektion einer Substanz anzugeben, die im Vergleich zum bekannten Stand der Technik eine höhere Massensensitivität für die Substanz aufweist.

- Zur Lösung der Aufgabe wird eine Vorrichtung zur Detektion mindestens einer Substanz eines Fluids angegeben. Die Vorrichtung weist mindestens einen piezoakustischen Resonator mit mindestens einer piezoelektrischen Schicht, einer an der piezoelektrischen Schicht angeordneten Elektrode, mindestens einer an der piezoelektrischen Schicht angeordneten weiteren Elektrode und einem Oberflächenabschnitt zur Sorption der Substanz des Fluids auf, wobei die piezoelektrische Schicht, die Elektroden und der Oberflächenabschnitt derart aneinander angeordnet sind, dass eine elektrische Ansteuerung der Elektroden zu einer Dickenschwingung des Resonators mit einer Resonanzfrequenz führt und die Resonanzfrequenz abhängig ist von einer am Oberflächenabschnitt sorbierten Menge der Substanz. Die Vorrichtung ist dadurch gekennzeichnet, dass eine Schichtdicke der piezoelektrischen Schicht aus dem Bereich von einschließlich 0,1 $\mu$m bis einschließlich 20 $\mu$m und die Resonanzfrequenz der Dickenschwingung aus dem Bereich von einschließlich 500 MHz bis einschließlich 10 GHz ausgewählt sind und der Oberflächenabschnitt zur Sorption der Substanz des Fluids von einer chemisch sensitiven Beschichtung des Resonators gebildet ist,der Resonator auf einem Halbleitersubstrat angeordnet ist und mindestens eine Einrichtung zur akustischen Isolation des Resonators und des Halbleitersubstrats vorhanden ist, dadurch gekennzeichnet, dass die Einrichtung zur akustischen Isolation ein im Substrat integrierter akustischer Spiegel ist.

Der Resonator der Vorrichtung ist ein piezoakustischer Dünnfilmresonator. Die Elektroden sind aus Elektrodenschichten gebildet. Die Elektrodenschichten bestehen beispielsweise aus Gold, Aluminium oder Platin. Eine Gesamtschichtdicke des Resonators aus Elektrodenschichten und piezoelektrischer Schicht beträgt beispielsweise 1 $\mu$m. Durch die kleine Schichtdicke der piezoelektrischen Schicht beziehungsweise durch die kleine Gesamtschichtdicke ist die Masse des Resonators im Vergleich zum Stand der Technik reduziert. Dadurch erhöht sich die Resonanzfrequenz des Resonators. Durch die hohe Resonanzfrequenz, die auch als Hochfrequenz bezeichnet wird, ergibt sich eine hohe Massensensitivität gegenüber der Substanz. Die Massensensitivität der Vorrichtung kann nach Gleichung (1) auf wenige Hz·pg$^{-1}$·cm$^2$ abgeschätzt werden. Beispielsweise beträgt die Massensensitivität bei einer Resonanzfrequenz von 1 GHz etwa 2,5 Hz·pg$^{-1}$·cm$^2$. Mit der Erfindung wird eine um einen Faktor von 10$^3$ verbesserte Massensensitivität erzielt.

Unter Sorption ist die Ausbildung einer chemischen oder physikalischen Bindung der Substanz an den Oberflächenabschnitt zu verstehen. Die Sorption umfasst dabei sowohl eine Absorption als auch eine Adsorption. Bei der Absorption wird die Substanz beispielsweise durch eine Beschichtung des Resonators, die den Oberflächenabschnitt bildet, ohne Bildung einer Phasengrenze aufgenommen. Die Substanz wird in die Beschichtung inkorporiert. Bei der Adsorption kommt es dagegen zur Bildung einer Phasengrenze. Insbesondere denkbar ist dabei eine Adsorption in Form einer Physisorption. Die Substanz lagert sich am Oberflächenabschnitt des Resonators durch Van der Waals- oder Dipol-Dipol-Wechselwirkungen an. Alternativ dazu kann auch eine Adsorption in Form einer Chemisorption stattfinden. Bei einer Chemisorption lagert sich die Substanz am Oberflächenabschnitt unter Bildung einer chemischen Bindung an. Die chemische Bindung ist beispielsweise eine kovalente Bindung oder eine Wasserstoffbrückenbindung.

Vorzugsweise findet die Sorption reversibel statt. Dies bedeutet, dass die Substanz vom Oberflächenabschnitt auch wieder desorbiert (entfernt) werden kann. Beispielsweise wird die Substanz durch Temperaturerhöhung des Oberflächenabschnitts oder durch Einwirken eines reaktiven Stoffes wieder entfernt. Der reaktive Stoff ist beispielsweise eine Säure oder eine Lauge, mit deren Hilfe die bei der Chemisorption gebildeten Bindungen gelöst werden. Die Vorrichtung kann auf diese Weise mehrmals benutzt werden. Möglich ist aber auch, dass die Sorption irreversibel ist. Die Vorrichtung wird als Einwegsensor nur einmalig verwendet.

Als Substanz kommt jede denkbare chemische oder biologische Verbindung in Frage. So kann die Vorrichtung als Gassensor zur Detektion eines Gases eingesetzt werden. Das Gas ist eine Substanz, die einen bestimmten Dampfdruck aufweist. Derartige Substanzen sind beispielsweise organische Lösungsmittel. Denkbar ist auch, dass eine derartige Substanz ein Sprengstoff oder ein Bestandteil, ein Vorprodukt oder ein Abbauprodukt eines Sprengstoffs ist. Die Vorrichtung kann als Sprengstoffdetektor eingesetzt werden. Denkbar ist auch, dass die Vorrichtung als Biosensor zur Detektion eines beliebigen Biomoleküls ausgestaltet ist. Das Biomolekül ist beispielsweise eine DNA(Deoxyribonucleic Acid)-Sequenz oder ein makromolekulares Protein.

Der Oberflächenabschnitt ist vorzugsweise derart ausgebildet, dass selektiv eine bestimmte Substanz oder Substanzklasse nach dem Schlüssel-Schloss-Prinzip sorbiert und damit erkannt wird. Somit ist es möglich, aus einem Gemisch aus einer Vielzahl von Substanzen mit Hilfe der Vorrichtung selektiv eine bestimmte Substanz zu detektieren. Die Detektion umfasst dabei sowohl eine qualitative als auch quantitative Bestimmung der Substanz. Es kann die Abwesenheit oder die Anwesenheit der Substanz im Fluid nachgewiesen werden. Es kann auch die Konzentration der Substanz im Fluid bestimmt werden. Durch differentielle Detektion der Substanz kann auch eine zeitliche Änderung der Konzentration der Substanz bestimmt werden. Somit eignet sich die Vorrichtung beispielsweise auch zur Reaktionskontrolle einer chemischen Reaktion, an der die Substanz beteiligt ist.

Insbesondere weist die chemisch sensitive Beschichtung Moleküle zum Erkennen der Substanz. Zum Erkennen einer bestimmten DNA-Sequenz sind derartige Moleküle entsprechende Oligo-Nukleotide (DNA-Oliogos) aus mehreren Nukleotid-Einheiten.

Die Moleküle zum Erkennen der Substanz können dabei direkt mit einer Transducer-Oberfläche verbunden sein. Beispielsweise ist die Transducer-Oberfläche eine Gold-Elektrode des Resonators. Moleküle, die über eine Thiol-Gruppe verfügen, werden durch Ausbilden einer Gold-Schwefel-Bindung direkt an die Transducer-Oberfläche gebunden.

In einer besonderen Ausgestaltung weist die chemisch sensitive Beschichtung eine Immobilisierungsschicht zum Verbinden des Resonators und der Moleküle zum Erkennen der Substanz auf. Beispielsweise verfügt eine Transducer-Oberfläche über NH- oder OH-Gruppen. Die Moleküle zum Erkennen der Substanz können dabei über Alkoxysilane, Cyanurchlorid oder Carbodiimid immobilisiert werden. Diese Verbindungen bilden die Immobilisierungsschicht.

Die Immobilisierungsschicht kann direkt mit der Transducer-Oberfläche verbunden sein. Denkbar ist auch, dass die Immobilisierungsschicht indirekt über eine Haftvermittlungsschicht mit der Transducer-Oberfläche verbunden ist.

Die Immobilisierungsschicht kann im Wesentlichen zweidimensional sein. Die Immobilisierungsschicht ist als geordnete monomolekulare oder multimolekulare Schicht entlang der Transducer-Oberfläche angeordnet. Insbesondere aber ist die Immobilisierungsschicht dreidimensional. Es liegt eine Immobilisierungsmatrix vor. Beispielsweise verfügt die Immobilisierungsschicht über offene Poren, in denen die Moleküle zum Erkennen der Substanz angeordnet sind. Es liegt eine chemisch sensitive Beschichtung mit einer großen "reaktiven" Oberfläche vor. Als Folge davon zeichnet sich eine chemisch sensitive Beschichtung mit einer dreidimensionalen Immobilisierungsschicht durch eine erhöhte Massensensitivität für das Erkennen der Substanz aus. Die dreidimensionale Immobilisierungsschicht kann beispielsweise durch radikalische Vernetzung von Monomeren erzeugt werden. An die vernetzten Monomere können die Moleküle zum Erkennen der Substanz gebunden werden. Denkbar ist auch, dass die Monomere bereits vor dem Vernetzen über die funktionellen Gruppen zum Erkennen der Substanz verfügen.

Insbesondere ist die Schwingung des Resonators aus der Gruppe Längsschwingung und/oder Dickenscherschwingung ausgewählt. Welche Schwingungsart angeregt wird, hängt unter anderem von einer Symmetriegruppe des piezoelektrischen Materials, der Orientierung der piezoelektrischen Schicht zur Oberfläche und von der Anordnung der Elektroden ab. Beispielsweise besteht die piezoelektrische Schicht aus einem <111> orientierten Bleizirkonattitanat. Wird ein elektrisches Feld nur in z-Richtung entlang der Schichtdicke der piezoelektrischen Schicht angelegt, so kommt es in erster Linie zu einer Längsschwingung entlang der Schichtdicke. Dagegen kann die Dickenscherschwingung bei der beschriebenen Anordnung entlang der lateralen Ausdehnung der piezoelektrischen Schicht auftreten. Die Dickenscherschwingung benötigt dazu allerdings eine laterale Komponente des anregenden elektrischen Feldes. Die Längsschwingung wird insbesondere zur Untersuchung eines gasförmigen Fluids eingesetzt. Bei einem flüssigen Fluid wird die Längsschwingung relativ stark gedämpft, wodurch die Massensensitivität stark reduziert wird. Zur Untersuchung eines flüssigen Fluids unter Ausnutzung der Längsschwingung des Resonators wird daher das Fluid nach der Sorption vom Oberflächenabschnitt beziehungsweise vom Resonator entfernt. Die Messung der Resonanzfrequenz des Resonators findet nach der Sorption in Abwesenheit des Fluids statt. Zur direkten Untersuchung eines flüssigen Fluids eignet sich dagegen die Messung der Dickenscherschwingung. Die Dickenscherschwingung wird in einer Flüssigkeit nur unmerklich gedämpft. Die Messung kann bei Flüssigkeitskontakt des Resonators erfolgen.

In einer besonderen Ausgestaltung weist der Resonator eine laterale Ausdehnung auf, die aus dem Bereich von einschließlich 50 μm bis einschließlich 1000 μm ausgewählt ist. Durch die kleine laterale Ausdehnung kann mit Hilfe des Resonators ein kleines Probenvolumen des Fluids untersucht werden.

In einer besonderen Ausgestaltung weist die piezoelektrische Schicht ein piezoelektrisches Material auf, das aus der Gruppe Bleizirkonattitanat, Zinkoxid und/oder Aluminiumnitrid ausgewählt ist. Diese Materialien eignen sich besonders für ein Abscheiden der Materialien aus der Gasphase auf einem Substrat. Das Abscheiden erfolgt beispielweise in einem chemischen Dampfabscheideverfahren (Chemical Vapour Deposition, CVD) oder einem physikalischen Dampfabscheidverfahren (Physical Vapour Depostion, PVD). Das physikalische Dampfabscheideverfahren ist beispielsweise Sputtern. Mit Hilfe der Dampfabscheideverfahren sind die kleine Schichtdicke der piezoelektrischen Schicht und auch kleine Schichtdicken der Elektroden zugänglich.

Der Resonator kann auf einem beliebigen Substraten angeordnet sein, das einen geringen Verlust für Hochfrequenzsignale aufweist. Dieses Substrat weist als Dielektrikum beispielsweise einen Saphir auf. Denkbar ist insbesondere ein Hochfrequenzsubstrat. Das Hochfrequenzsubstrat zeichnet sich dadurch aus, dass ein Hochfrequenzsignal mit einer hohen Güte und damit mit einem geringen Verlust weitergeleitet wird. Als Hochfrequenzsubstrat kommt insbesondere ein LTCC-Substrat (Low Temperature Cofired Ceramics) zur Anwendung. Im LTCC-Substrat können aufgrund der Verwendung von bei niedriger Temperatur sinternder Glaskeramik elektrisch hochleitfähige Materialien wie metallisches Kupfer oder Silber integriert sein.

Insbesondere ist der Resonator auf einem Halbleitersubstrat angeordnet. Das Halbleitersubstrat weist dabei insbesondere ein Halbleitermaterial auf, das aus der Gruppe Silizium und/oder Galliumarsenid ausgewählt ist. Diese Halbleitermaterialien eignen sich zur Anwendung der Bipolar- und CMOS-Technologie. Mit Hilfe dieser Technologien lässt sich im Halbleitersubstrat ein Schaltkreis, beispielsweise ein Auswerteschaltkreis zur Bestimmung der Resonanzfrequenz des Resonators integrieren. Es resultiert eine hohe Integrationsdichte.

In einer besonderen Ausgestaltung der Vorrichtung ist mindestens eine Einrichtung zur akustischen Isolation des Resonators und des Halbleitersubstrats vorhanden. Der Resonator und das Halbleitersubstrat sind akustisch voneinander isoliert. Durch die akustische Isolation des Resonators und des Halbleitersubstrats ist gewährleistet, dass die Resonanzfrequenz des Resonators unabhängig vom Halbleitersubstrat ist. Es resultiert eine relativ hohe Massensensitivität. Die Einrichtung zur akustischen Isolation ist beispielsweise ein im Substrat integrierter akustischer Spiegel. Der akustische Spiegel ist beispielsweise ein akustischer Bragg-Reflektor, der aus λ/4-dicken Schichten unterschiedlicher akustischer Impedanz besteht. Alternativ dazu wird die Einrichtung durch einen Hohlraum im Substrat gebildet, der durch eine Membran abgedeckt ist. Der Resonator ist über die Membran (mittelbar) mit dem Halbleitersubstrat verbunden. Die Membran besteht beispielsweise aus einem Oxid und/oder Nitrid. Beispielsweise ist die Membran eine Mehrschichtmembran aus einer OxidSchicht und einer Nitrid-Schicht. Zur akustischen Isolation ist es auch möglich, dass eine dem Resonator abgekehrte Rückseite des Halbleitersubstrats eine Ausnehmung aufweist. Die Ausnehmung wird vorzugsweise durch Rückseitenätzung des Halbleitersubstrats hergestellt. Der Resonator ist beispielsweise auf einer durch die Ausnehmung freistehenden Membran aus dem Nitrid mit dem Halbleitersubstrat verbunden.

In einer besonderen Ausgestaltung ist der Oberflächenabschnitt zur Sorption der Substanz des Fluids an einer Ausnehmung des Halbleitersubstrats angeordnet. Die Ausnehmung, die beispielsweise als Einrichtung zur akustischen Isolation des Resonators und des Halbleitersubstrats ausgestaltet sein kann, weist den Oberflächenabschnitt zur Sorption der Substanz auf. Der Oberflächenabschnitt kann dabei von einer chemisch sensitiven Beschichtung des Resonators gebildet sein, die in der Ausnehmung angeordnet und für das Fluid zugänglich ist. Denkbar ist auch, dass der Oberflächenabschnitt zumindest teilweise von einer Elektrode oder von der piezoelektrischen Schicht des Resonators gebildet ist, die im Bereich der Ausnehmung für das Fluid zugänglich sind.

In einer weiteren Ausgestaltung der Vorrichtung ist mindestens eine Auswerteeinrichtung vorhanden zur Bestimmung der Resonanzfrequenz des Resonators. Die Auswerteeinrichtung umfasst beispielsweise einen Hochfrequenzschaltkreis, in den der Resonator als frequenzbestimmendes Bauteil eingebunden ist. Der Hochfrequenzschaltkreis ist beispielsweise eine PLL (Phased Locked Loop), in dessen Regelschleife über PIN-Dioden der Resonator zugeschaltet wird. Das Regelsignal der PLL ist ein Maß für die Änderung der Resonanzfrequenz des Resonators. Durch die Änderung der Resonanzfrequenz kann auf die Sorption der Substanz am Oberflächenabschnitt des Resonators geschlossen werden. Insbesondere ist die Auswerteeinrichtung eine im Halbleitersubstrat angeordnete interne Auswerteeinrichtung. Die interne Auswerteeinrichtung lässt sich auf bekannte Weise in Bipolar- oder in CMOS-Technologie im Halbleitersubstrat integrieren. Durch die Möglichkeit, die Auswerteinrichtung im Halbleitersubstrat zu integrieren, kann die Vorrichtung als Modul eines sogenannten "lab on a chip" fungieren.

Alternativ dazu ist die Auswerteeinrichtung eine außerhalb des Halbleitersubstrats angeordnete externe Auswerteeinrichtung. Die Auswerteeinrichtung ist beispielsweise ein für die Auswertung der Resonanzfrequenz ausgestalteter Auswertechip. Der Auswertechip ist beispielsweise zusätzlich auf dem Halbleitersubstrat aufgebracht und mit dem Resonator elektrisch kontaktiert.

In einer weiteren Ausgestaltung ist mindestens eine Einrichtung zur elektrischen Kontaktierung des Resonators und der externern Auswerteeinrichtung vorhanden, die ein aus der Gruppe FR4-Substrat und/oder LTCC-Substrat ausgewähltes Hochfrequenzsubstrat ist. Beispielsweise ist die externe Auswerteeinrichtung auf einem LTCC-Substrat aufgebracht und mit Hilfe des LTCC-Substrats mit dem Resonator elektrisch verbunden. FR4 ist ein bekanntes Hochfrequenzmaterial. Denkbar sind auch Substrate aus andern Hochfrequenzmaterialien wie GETEK oder RO4003.

In einer besonderen Ausgestaltung ist der Resonator mit Halbleitersubstrat und das Hochfrequenzsubstrat mit einer Flip-Chip-Technik miteinander verbunden. Die Flip-Chip-Technik ist besonderes platzsparend. Elektrische Kontaktierungen werden in vertikaler Richtung mit Hilfe sogenannter Löt-Bumps erzeugt. Die Löt-Bumps sind kleine Erhebungen, beispielsweise Kügelchen, aus einem Lot auf der Substratoberfläche. Die Löt-Bumps bestehen beispielsweise aus einer Gold-Nickel- oder Gold-Zinn-Legierung. Durch die Löt-Bumps sind Platz verbrauchende Verbindungsdrähte (Bonddrähte) nicht nötig. Durch die Platzeinsparung kann die Miniaturisierung der Vorrichtung weiter vorangetrieben werden. Besonders vorteilhaft daran ist, dass ein störender Einfluss von Induktivitäten der Bonddrähte weitgehend eliminiert werden kann. Zudem werden unnötig lange elektrische Leitungen auf dem Halbleitersubstrat vermieden. Insbesondere bei Frequenzen im GHz-Bereich zeichnen sich Leitungen auf einem Halbleitersubstrat durch große innere Verluste aus. Durch die Anwendung der Flip-Chip-Technik kann auf solche Leitungen auf dem Halbleitersubstrat weitgehend verzichtet werden. Die Vorrichtung zur Detektion einer Substanz zeichnet sich daher durch eine besonders hohe Betriebsgüte und in Folge davon auch durch eine besonders hohe Massensensitivität aus.

Die Anwendung der Flip-Chip-Technik ist insbesondere mit der Ausgestaltung der Vorrichtung vorteilhaft, bei der der Oberflächenabschnitt zur Sorption der Substanz in der Ausnehmung des Halbleitersubstrats angeordnet ist. Eine geätzte Rückseite des Halbleitersubstrats mit dem Oberflächenabschnitt wird zur Sorption der Substanz des Fluids genutzt. Die der Rückseite abgekehrte Seite mit dem Resonator dient der elektrischen Kontaktierung der Elektroden des Resonators. Die Elektroden des Resonators werden über Löt-Bumps mit einem eventuell vorhandenen Hochfrequenzsubstrat elektrisch und mechanisch kontaktiert.

Der Oberflächenabschnitt zur Sorption der Substanz kann von einer der Elektroden des Resonators gebildet sein. In einer besonderen Ausgestaltung wird der Oberflächenabschnitt zur Sorption der Substanz des Fluids von einer chemisch sensitiven Beschichtung des Resonators gebildet. Auf dem Resonator ist eine Beschichtung aufgebracht, die eine bestimmte Substanz oder auch eine bestimmte Substanzklasse sorbieren kann. Die Sorption erfolgt über Chemisorption, Physisorption oder auch Absorption. Die chemisch sensitive Beschichtung ist beispielsweise ein eingangs genanntes Polymer. Denkbar ist auch, dass die Beschichtung an der Oberfläche eine bestimmte DNA-Sequenz aufweist. An diese DNA-Sequenz kann die korrespondierende DNA-Sequenz nach dem Schlüssel-Schloss-Prinzip andocken. Die korrespondierende DNA-Sequenz wird chemisorbiert unter Bildung von Wasserstoffbrückenbindungen. Die Vorrichtung eignet sich in dieser Form zum Nachweis einer bestimmten DNA-Sequenz. Denkbar ist auch eine chemisch sensitive Beschichtung für beliebige chemische und biologische Substanzen.

Die chemisch sensitive Beschichtung kann aber auch zur Adsorption bestimmter gasförmiger Moleküle ausgestaltet sein. Derartige Moleküle sind beispielsweise Kohlenmonoxid, Kohlendioxid, Stickoxide oder Schwefeloxide. Niedermolekulare organische Gase wie Methan oder Ethan sind ebenfalls denkbar. Die Vorrichtung wird zur Gassensorik verwendet.

In einer besonderen Ausgestaltung ist eine Vielzahl von Resonatoren zu einer Resonatormatrix zusammengefasst und jeder der Resonatoren bildet ein Matrixelement der Resonatormatrix. Aufgrund der kleinen lateralen Ausdehnung jedes einzelnen Resonators können im Vergleich zum Stand der Technik pro Flächeneinheit wesentlich mehr Resonatoren zur Resonatormatrix zusammengefasst werden. Vorzugsweise dient dabei jeder der Resonatoren der Resonatormatrix der Detektion einer bestimmten Substanz. Dies bedeutet, dass jeder Resonator derart ausgelegt ist, dass er für eine bestimmte Substanz oder Substanzklasse sensitiv ist. Auf diese Weise können mit Hilfe der Vorrichtung Substanzgemische qualitativ und/oder quantitativ analysiert werden. Bei einem Gassensor ist beispielsweise einer der Resonatoren sensitiv für Kohlenwasserstoffe, ein zweiter Resonator sensitiv für Schwefeloxide und ein weiterer Resonator sensitiv für Kohlenmonoxid. Bei einem Biosensor ist beispielsweise jeder der Resonatoren der einzelnen Matrixelemente sensitiv für eine bestimmte DNA-Sequenz. Somit lässt sich das Vorhandensein verschiedenen DNA-Sequenzen im Fluid parallel untersuchen.

Insbesondere ist die Resonatormatrix auf einem Halbleitersubstrat ausgebildet. Vorzugsweise ist die Resonatormatrix in Dünnfilmtechnologie auf dem Halbleitersubstrat hergestellt. In der Dünnfilmtechnologie werden die einzelnen Resonatoren in denselben Arbeitsschritten parallel hergestellt. Durch die Anwendung der Dünnfilmtechnologie ist eine Streuung bezüglich der elektrischen Eigenschaften benachbarter Resonatoren gering. Wird beispielsweise ein Resonator mit einer chemisch sensitiven Beschichtung versehen und ein benachbarter Resonator bleibt dagegen unbeschichtet, so kann die Änderung der Resonanzfrequenz des beschichteten Resonators gegenüber der Resonanzfrequenz des unbeschichteten Resonators zur Detektion der Substanz herangezogen werden. Eine differentielle Messung ist möglich.

Vorzugsweise ist ein Abstand zwischen benachbarten Matrixelementen aus dem Bereich von einschließlich 20 $\mu$m bis einschließlich 1000 $\mu$m ausgewählt. Es resultiert eine Vorrichtung mit einer Resonatormatrix, in der auf einem möglichst kleinen Raum möglichst viele Matrixelemente integriert sind. Jedes der Matrixelemente ist sehr klein. Es resultiert daher eine Vorrichtung mit sehr kleinem lateralen Gesamtausmaß.

Gemäß einem zweiten Aspekt der Erfindung wird ein Verfahren zur Detektion mindestens einer Substanz eines Fluids unter Verwendung der beschriebenen Vorrichtung angegeben. Das Verfahren beinhaltet die Verfahrensschritte Zusammenbringen des Fluids und des piezoakustischen Resonators derart, dass die Substanz am Oberflächenabschnitt des Resonators sorbiert werden kann und Bestimmen einer Resonanzfrequenz des Resonators, wobei aus der Resonanzfrequenz auf die am Oberflächenabschnitt sorbierte Menge der Substanz geschlossen wird. Um auf die sorbierte Menge der Substanz schließen zu können, wird in der Regel vor dem Zusammenbringen des Fluids und des Resonators die Resonanzfrequenz des Resonators ohne sorbierte Substanz bestimmt.

Vorzugsweise wird die Resonanzfrequenz nach dem Zusammenbringen des Fluids und des Resonators in Gegenwart des Fluids bestimmt. Beispielsweise ist das Fluid ein Gas bzw. Gasgemisch. Das Gasgemisch wird am Oberflächenabschnitt eines oder mehrerer Resonatoren vorbeigeleitet. Die Substanz wird am Oberflächenabschnitt sorbiert. Durch die Sorption ändert sich die Masse des Resonators und damit die Resonanzfrequenz des Resonators. Je mehr Substanz sorbiert wird, desto größer ist die Änderung der Resonanzfrequenz im Vergleich zum Resonator, an dessen Oberflächenabschnitt keine Substanz adsorbiert ist.

Gemäß einer weiteren Ausgestaltung wird die Resonanzfrequenz in Abwesenheit des Fluids bestimmt. Beispielsweise wird das Fluid in einem ersten Schritt am Oberflächenabschnitt des Resonators vorbeigeleitet. Es findet die Sorption statt. Nach beendeter Sorption wird das Vorbeileiten des Fluids beendet. Danach wird das Fluid derart entfernt, dass die Substanz am Oberflächenabschnitt des Resonators sorbiert bleibt. Nachfolgend wird die Resonanzfrequenz des Resonators bestimmt. Dieses Verfahren wird insbesondere dann eingesetzt, wenn als Fluid eine Flüssigkeit und als Schwingung die Längsschwingung des Resonators verwendet wird. Im Übrigen ist auch denkbar, dass die Resonanzfrequenz sowohl in Gegenwart als auch in Abwesenheit des Fluids bestimmt wird.

Zusammenfassend ergeben sich mit der Erfindung folgende Vorteile:

- Die Vorrichtung zur Detektion einer Substanz zeichnet sich durch eine im Vergleich zum Stand der Technik verbesserte Massensensitivität aus. Die Massensensitivität beträgt wenige Hz·pg$^{-1}$·cm$^2$.

- Durch Verwendung einer dreidimensionalen Immobilisierungsmatrix für Moleküle zum Erkennen der Substanz kann die Massensensitivität zusätzlich erhöht werden.

- Die hohe Massensensitivität führt dazu, dass die Substanz (Analytmolekül) zur Detektion nicht markiert werden muss. Die Detektion erfolgt "labelfrei".

- Der Resonator der Vorrichtung zeichnet sich durch eine kleine laterale Ausdehnung aus. Aufgrund der kleinen lateralen Ausdehnung kann eine Vielzahl von Resonatoren platzsparend auf einem Substrat zu einer Resonatormatrix angeordnet werden. Es resultiert eine hohe Integrationsdichte.

- Mit Hilfe der Resonatormatrix kann eine Vielzahl von Substanzen parallel detektiert werden.

- Wegen der kleinen lateralen Ausdehnung des Resonators kann ein kleines Probenvolumen des Fluids untersucht werden.

- Die Vorrichtung mit dem Resonator ist einfach und kostengünstig herzustellen. Zur Herstellung kann auf bekannte Dünnfilmtechnologien zurückgegriffen werden. Unter Verwendung eines Halbleitersubstrats sind CMOS-Technologie und bulk micromachining anwendbar.

- Zur elektrischen Kontaktierung des Resonators bzw. der Resonatoren eignet sich insbesondere ein Hochfrequenzsubstrat in Form eines LTCC-Substrats.

- Durch die Flip-Chip-Technik werden Hochfrequenzverluste und störende Induktivitäten weitgehend eliminiert, die in Bonddrähten zur elektrischen Kontaktierung des Resonators auftreten können. Es resultiert eine hohe Betriebsgüte und eine hohe Massensensitivität. Dies gilt insbesondere für eine Resonatormatrix mit einer hohen Dichte an Matrixelementen.

Anhand mehrerer Ausführungsbeispiele und der dazugehörigen Figuren werden die Vorrichtung und das Verfahren zur Detektion einer Substanz eines Fluids vorgestellt. Im Fokus stehen dabei verschiedene Einzelaspekte der Erfindung, die beliebig miteinander kombiniert werden sind. Die Figuren sind schematisch und stellen keine maßstabsgetreuen Abbildungen dar.

Figuren 1a bis 1f zeigen piezoakustische Resonatoren im Querschnitt, bei denen die Elektroden, die piezoelektrische Schicht und der Oberflächenabschnitt des Resonators unterschiedlich zueinander angeordnet sind.

Figuren 2a bis 2c zeigen unterschiedliche Möglichkeiten zur akustischen Isolation eines Halbleitersubstrats und des piezoakustischen Resonators.

Figuren 3a und 3b zeigen Vorrichtungen mit interner und externer Auswerteeinrichtung.

Figuren 4a bis 4c zeigen eine Resonatormatrix mit mehreren Matrixelementen im Querschnitt (Figur 4a) und in Aufsicht (Figuren 4b und 4c).

Figuren 5a und 5b deuten verschiedene Schwingungsmoden des Resonators an.

Figur 6 zeigt ein Verfahren zur Detektion einer Substanz eines Fluids.

Die Vorrichtung 1 zur Detektion einer Substanz besteht aus einem piezoakustischen Resonator 2, der auf einem Halbleitersubstrat 3 aus Silizium aufgebracht ist. Der Resonator 2 weist eine piezoelektrische Schicht 4 aus Bleizirkonattitanat. Das Bleizirkonattitanat weist eine <111> -Orientierung bezüglich des Halbleitersubstrats 3 auf. Die Schichtdicke 7 der piezoelektrischen Schicht 4 beträgt ca. 0,8 $\mu$m. Die laterale Ausdehnung 11 des Resonators 2 beträgt ca. 100 $\mu$m. In zwei dazu alternativen Ausführungen besteht die piezoelektrische Schicht aus Aluminiumnitrid und Zinkoxid.

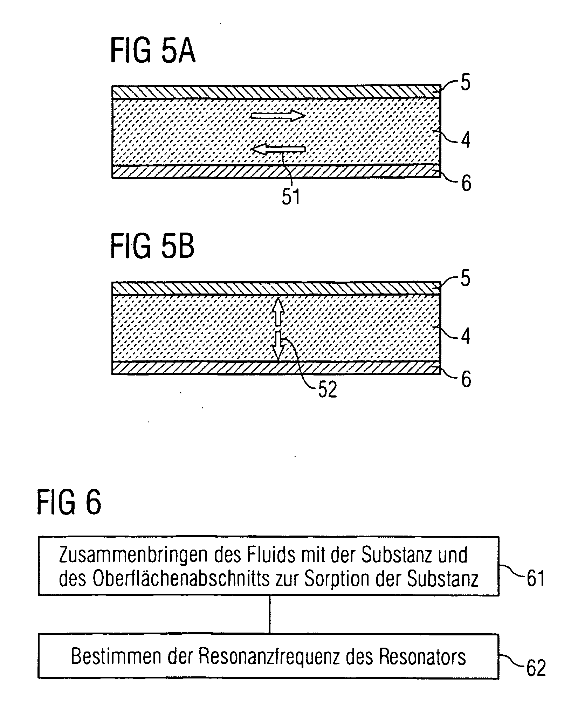

Die Schichtdicken der an der piezoelektrischen Schicht 4 angeordneten schichtförmigen Elektroden 5 und 6 betragen ca. 0,1 $\mu$m. Die Elektroden sind aus Gold. Durch elektrische Ansteuerung der Elektroden 5 und 6 wird der Resonator 2 zu einer Schwingung angeregt. In Abhängigkeit von der Anordnung der piezoelektrischen Schicht 4 und der Elektroden 5 und 6 zueinander wird der Resonator 2 zu einer Dickenscherschwingung 51 entlang einer lateralen Ausdehnung 11 der piezoelektrischen Schicht 4 (Figur 5a) und/oder zu einer Längsschwingung 52 entlang der Schichtdicke 7 der piezoelektrischen Schicht 4 (Figur 5b) angeregt.

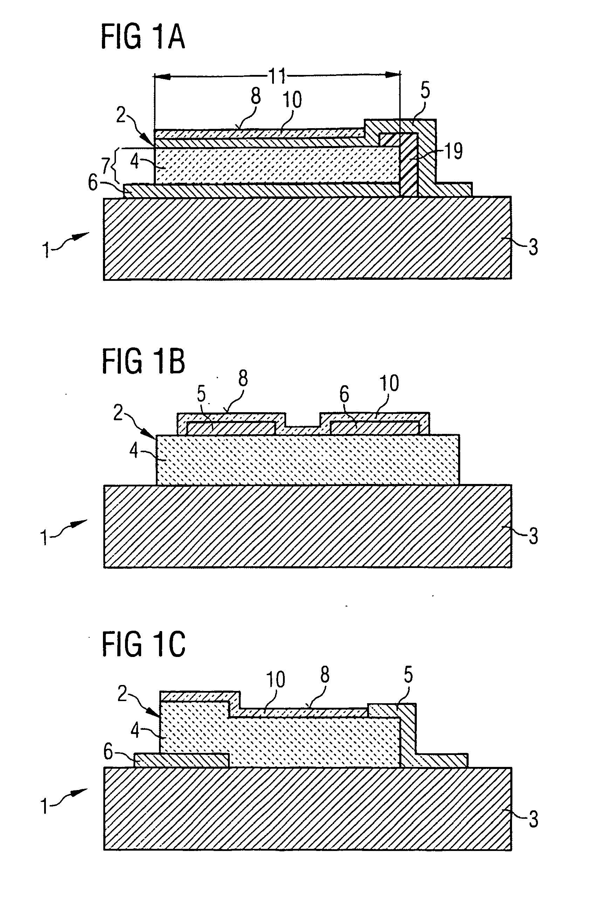

Gemäß Figur 1a sind die Elektroden 5 und 6 an zwei voneinander abgekehrten Seiten der piezoelektrischen Schicht 4 angeordnet. Eine elektrische Isolierung 19 aus Aluminiumoxid trennt die Elektroden 5 und 6 zusätzlich. Die elektrische Ansteuerung führt zu einer Längsschwingung 52 entlang der Schichtdicke 7 der piezoelektrischen Schicht 4.

Eine Anordnung gemäß Figuren 1b, bei der auf einer Seite der piezoelektrischen Schicht 4 beide Elektroden 5 und 6 angeordnet sind, führt zu einer Dickenscherschwingung 51. Ebenso führt die Anordnung gemäß Figur 1c zur Ausbildung einer Dickenscherschwingung 51.

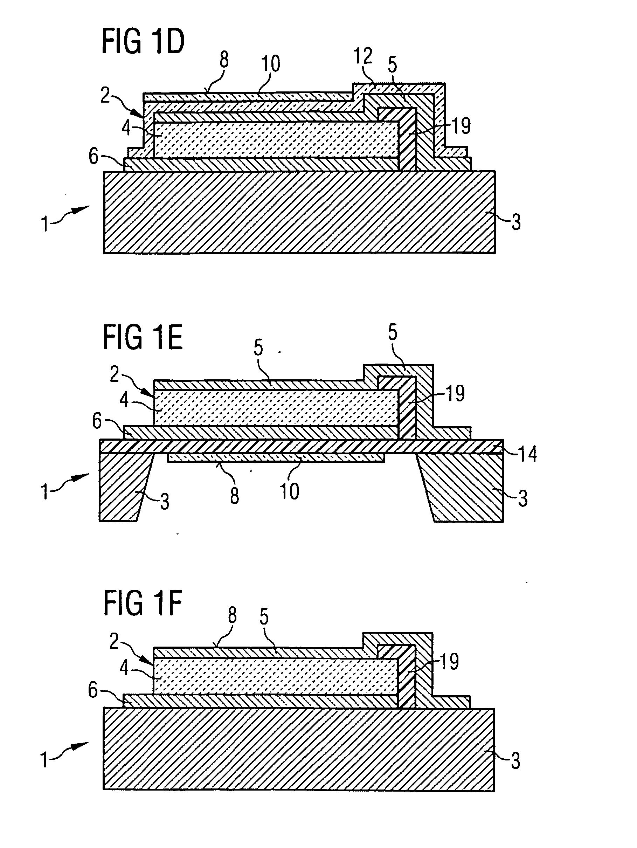

Der Resonator 2 verfügt über einen Oberflächenabschnitt 8, an dem eine Substanz eines Fluids 9 sorbiert werden kann. Dazu verfügt der Resonator 2 über eine chemisch sensitive Beschichtung 10. Die chemisch sensitive Beschichtung 10 ist auf der Elektrode 5 angebracht (Figur 1a). Alternativ dazu ist die chemisch sensitive Beschichtung auf beiden Elektroden 5 und 6 angebracht (Figur 1b). Die chemisch sensitive Beschichtung 10 kann auch auf der piezoelektrischen Schicht 4 aufgebracht sein (Figur 1c). Alternativ dazu ist auf dem Resonator 4 eine Schutzschicht 12 aufgebracht, auf die wiederum die chemisch sensitive Beschichtung 10 aufgebracht ist (Figur 1d). In einer nicht dargestellten Ausführungsform fungiert die Schutzschicht 12 selbst als chemisch sensitive Beschichtung 10.

Figur 1e zeigt eine weitere Alternative, gemäß der die chemisch sensitive Beschichtung 10 in einer Ausnehmung 13 des Halbleitersubstrats 3 auf einer Membran 14 aus einem Nitrid angeordnet ist. Die Ausnehmung 13 ist durch Rückseitenätzung des Halbleitersubstrats 3 hergestellt.

Gemäß Figur 1f bildet entgegen der vorangegangenen Beispiele die Elektrode 5 den Oberflächenabschnitt 8 zur Sorption der Substanz des Fluids 9. Die Substanz wird direkt auf der Elektrode sorbiert.

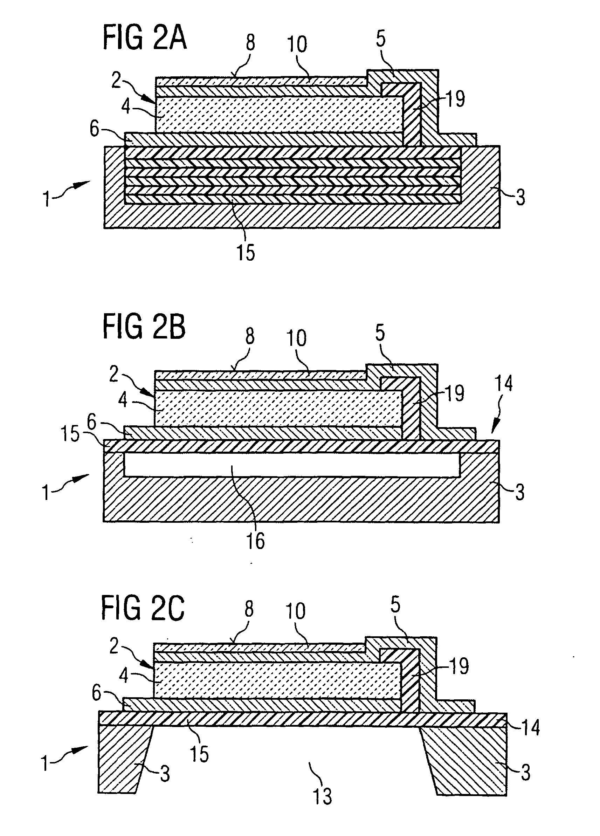

Um die Massensensitivität des Resonators 2 für eine bestimmte Substanz zu erhöhen, werden das Halbleitersubstrat 3 und der Resonator 2 mit Hilfe einer Einrichtung zur akustischen Isolation 15 akustisch voneinander isoliert. Gemäß Figur 2a ist die Einrichtung ein Bragg-Reflektor mit $\lambda/4$-dicken Schichten unterschiedlicher Impedanz. Alternativ dazu ist im Halbleitersubstrat 3 unterhalb des Resonators 2 eine Membran 14 und ein Hohlraum 16 integriert. Der Hohlraum 16 ist durch die Membran 14 abgedeckt. Die Membran 14 verbindet den Resonator 2 und das Halbleitersubstrat 3. Membran 14 und Hohlraum 16 sind über bulk- und/oder surface micromachining im Halbleitersubstrat realisiert.

Gemäß Figur 2c ist die Einrichtung 15 zur akustischen Isolation eine Ausnehmung 13 des Halbleitersubstrats 3. Unterhalb des Resonators 2 ist das Material des Halbleitersubstrats 3 durch Rückseitenätzung entfernt. Der Resonator 2 ist auf einer Membran 14 aufgebracht. Die Membran 14 besteht aus einer Siliziumnitrid-Schicht und einer Siliziumoxid-Schicht.

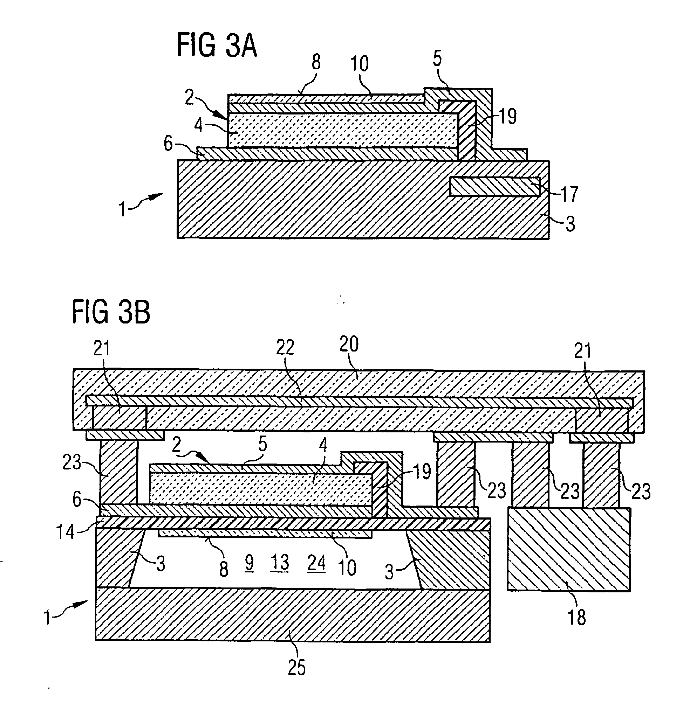

Gemäß Figur 3a ist eine interne Auswerteeinrichtung 17 im Halbleitersubstrat vorhanden. Die interne Auswerteeinrichtung 17 ist mit Hilfe der CMOS-Technologie im Halbleitersubstrat 3 integriert. Die interne Auswerteeinrichtung 17 umfasst einen Resonanzkreis, in den der Resonator 2 als Resonanz bestimmendes Bauelement eingebunden ist.

Gemäß Figur 3b ist der Resonator 2 mit einer externen Auswerteeinrichtung 18 verbunden. Zur Kontaktierung des Resonators 2 und der Auswerteeinrichtung 18 ist ein Hochfrequenzsubstrat 20 in Form eines LTCC-Substrats vorhanden. Im LTCC-Substrat sind elektrische Durchkontaktierungen 21 und elektrische Leiterbahnen 22 aus Silber integriert. Sowohl die Vorrichtung 1 als auch die externe Auswerteeinrichtung 18 werden mit Hilfe der Flip-Chip-Technik über Löt-Bumps 23 mit den im LTCC-Substrat 20 integrierten elektrischen Durchkontaktierungen 21 Leiterbahnen 22 elektrisch kontaktiert. Gemäß der Ausgestaltung nach Figur 3b ist die chemisch sensitive Beschichtung 10 in der Ausnehmung 13 des Halbleitersubstrats 3 angeordnet. Die Ausnehmung 13 fungiert als Kanal 24, durch den das Fluid 9 aus einem Reservoir zum Oberflächenabschnitt 8 zugeleitet wird. Der Kanal 24 wird von der Ausnehmung 13 des Halbleitersubstrats 3 und einer mit dem Halbleitersubstrat 3 verbundenen Abdeckung 25 gebildet. Die Abdeckung 25 ist ebenfalls aus Silizium. In einer dazu alternativen Ausgestaltung ist die Abdeckung 25 aus einem Kunststoff. Der Kunststoff und eine eventuell vorhandene Dichtung zwischen Kunststoff und Halbleitersubstrat ist gegenüber dem Fluid 9 reaktionsträge. Das Fluid 9 kann durch den Kanal 24 geleitet werden, ohne dass es zu einer Reaktion zwischen einem Bestandteil des Fluids 9 und dem Kunststoff oder einem Material der Dichtung kommt.

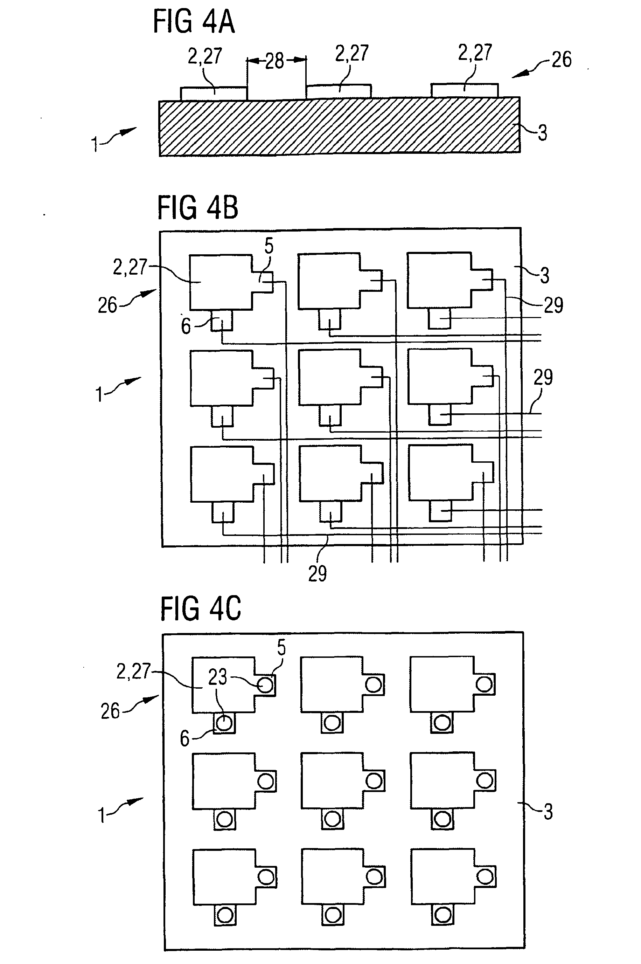

Figur 4a zeigt eine Vorrichtung 1 mit drei Resonatoren 2 im einem seitlichen Querschnitt entlang der Verbindungslinie I-I (siehe Figuren 4b und 4c). Die Resonatoren 2 sind auf einem Halbleitersubstrat 3 aus Silizium zu einer Resonatormatrix 26 aufgebracht. Die Resonatoren 2 bilden je ein Matrixelement 27 der Resonatormatrix 26. Jeder der Resonatoren 2 weist eine laterale Ausdehnung 11 von etwa 100 $\mu$m auf. Ein Abstand 28 zwischen benachbarten Matrixelementen 27 beträgt 100 $\mu$m.

Figuren 4b und 4c zeigen unterschiedliche Möglichkeiten der elektrischen Kontaktierung der einzelnen Matrixelemente 26. Gemäß Figur 4b werden die Elektroden 5 und 6 der Resonatoren 2 der einzelnen Matrixelemente 27 in herkömmlicher Weise elektrisch kontaktiert. Dazu sind auf dem Halbleitersubstrat 3 entsprechende elektrische Leitungen 29 vorhanden.

Gemäß Figur 4c erfolgt die elektrische Kontaktierung der einzelnen Resonatoren 2 über Löt-Bumps 23 in Flip-Chip-Technik.

Jedes der Matrixelemente 27 verfügt über einen für eine bestimmte Substanz sensitiven Resonator 2. Es liegt eine Vorrichtung zur Detektion einer Vielzahl von Substanzen eines Fluids 9 vor.

Zur Detektion der Substanzen des Fluids 9 werden jeweils in einem ersten Schritt der Oberflächenabschnitt 3 des Resonators 3 und das Fluid 9 zusammengebracht (Figur 6, Schritt 61). Fluid 2 und Resonator 2 werden derart zusammengebracht, dass die Substanz des Fluids 9 auf den jeweiligen Oberflächenabschnitten 3 des Resonators 2 sorbiert werden kann. Durch die Sorption ändert sich die Masse des Resonators 2. Durch nachfolgende Messung der Resonanzfrequenzen des Resonators 2 (Figur 6, Schritt 62) kann auf die Art der Substanz und deren Konzentration im Fluid 9 geschlossen werden. Durch die Sorption der Substanz verändert sich die Resonanzfrequenz des Resonators im Vergleich zur Resonanzfrequenz des Resonators, an dessen Oberflächenabschnitt keine Substanz sorbiert ist. Um die Änderung der Resonanzfrequenz bestimmen zu können, wird ein Resonator mit bekannter Resonanzfrequenz verwendet. In einer alternativen Ausführung wird vor dem Zusammenbringen des Fluids und des Resonators die Resonanzfrequenz des Resonators ohne sorbierte Substanz bestimmt.

Beispiel Protein-Detektion:

Auf der Elektrode 5 (aus Gold) des piezoakustischen Resonators 2 wird eine chemisch sensitive Beschichtung 10 aus einem Oligo-Nukleotid aus 25 Basen immobilisiert. Das Oligo-Nukleotid wird mit einer Konzentration von wenigen mmol im sub-Nanoliterbereich als wässrige Lösung auf der Elektrode 5 aufgetragen. Jedes der Oligo-Nukleotide weist an der 3'-Position eine Thiol-Alkyl-Gruppe und an der 5'-Position eine Biotin-Gruppe auf. Über die Thiol-Alkyl-Gruppe kommt es zur Ausbildung von Schwefel-Gold-Bindungen. Die Oligo-Nukleotide werden auf der Elektrode 5 immobilisiert.

Das Oligo-Nukleotid-Grundgerüst bildet quasi eine Immobilisierungsschicht. Die Biotin-Gruppe bildet einen starken Komplex mit Streptavidin. Die Biotin-Gruppe fungiert quasi als Molekül zum Erkennen der Substanz Streptavidin. Sobald dieses Protein in einem Fluid vorhanden ist, dem die beschriebene chemisch sensitive Beschichtung 10 ausgesetzt ist, kommt es zur Komplexbildung und damit zur Sorption des Proteins auf der chemisch sensitiven Beschichtung 10.

Beispiels DNA-Detektion:

Es werden Oligo-Nukleotide aus 25 Basen über Thiol-Alkylgruppen immobilisiert. Die Oligo-Nukleotide weisen keine Biotin-Gruppen auf. DNA-Fragmente mit einer entsprechend komplementären Nukleotid-Sequenz werden über die Ausbildung von Wasserstoff-Brücken-Bindungen an die immobilisierten Oligo-Nukleotide gebunden.

**Patentansprüche**

1. Vorrichtung (1) zur Detektion mindestens einer Substanz eines Fluids (9), aufweisend mindestens einen piezoakustischen Resonator (2) mit

- mindestens einer piezoelektrischen Schicht (4),
- einer an der piezoelektrischen Schicht (4) angeordneten Elektrode (5, 6),
- mindestens einer an der piezoelektrischen Schicht (4) angeordneten weiteren Elektrode (6, 5) und
- einem Oberflächenabschnitt (8) zur Sorption der Substanz des Fluids (9),
- wobei die piezoelektrische Schicht (4), die Elektroden (5, 6) und der Oberflächenabschnitt (8) derart aneinander angeordnet sind, dass eine elektrische Ansteuerung der Elektroden (5, 6) zu einer Dickenschwingung des Resonators (2) mit einer Resonanzfrequenz führt und die Resonanzfrequenz abhängig ist von einer am Oberflächenabschnitt (8) sorbierten Menge der Substanz,
- eine Schichtdicke (7) der piezoelektrischen Schicht (4) aus dem Bereich von einschließlich 0,1 $\mu$m bis einschließlich 20 $\mu$m und
- die Resonanzfrequenz der Dickenschwingung aus dem Bereich von einschließlich 500 MHz bis einschließlich 10 GHz ausgewählt sind,
- der Oberflächenabschnitt (8) zur Sorption der Substanz des Fluids (9) von einer chemisch sensitiven Beschichtung (10) des Resonators (2) gebildet ist,
- der Resonator (2) auf einem Halbleitersubstrat (3) angeordnet ist und
- mindestens eine Einrichtung (15) zur akustischen Isolation des Resonators (2) und des Halbleitersubstrats (3) vorhanden ist,
**dadurch gekennzeichnet, dass**
- die Einrichtung zur akustischen Isolation ein im Substrat integrierter akustischer Spiegel ist.

2. Vorrichtung nach Anspruch 1, wobei die chemisch sensitive Beschichtung Moleküle zum Erkennen der Substanz aufweist.

3. Vorrichtung nach Anspruch 2, wobei die chemisch sensitive Beschichtung eine Immobilisierungsschicht zum Verbinden des Resonators und der Moleküle zum Erkennen der Substanz aufweist.

4. Vorrichtung nach einem der Ansprüche 1 bis 3, wobei der Resonator (2) eine laterale Ausdehnung (11) aufweist, die aus dem Bereich von einschließlich 20 $\mu$m bis einschließlich 1000 $\mu$m ausgewählt ist.

5. Vorrichtung nach einem der Ansprüche 1 bis 4, wobei die Dickenschwingung des Resonators (2) aus der Gruppe Längsschwingung (52) und/oder Dickenscherschwingung (51) ausgewählt ist.

6. Vorrichtung nach einem der Ansprüche 1 bis 5, wobei die piezoelektrische Schicht (4) ein piezoelektrisches Material aufweist, das aus der Gruppe Bleizirkonattitanat, Zinkoxid und/oder Aluminiumnitrid ausgewählt ist.

7. Vorrichtung nach einem der Ansprüche 1 bis 6, wobei der Oberflächenabschnitt (8) zur Sorption der Substanz des Fluids an einer Ausnehmung (13) des Halbleitersubstrats (3) angeordnet ist.

8. Vorrichtung nach einem der Ansprüche 1 bis 7, wobei mindestens eine Auswerteeinrichtung (17, 18) vorhanden ist zur Bestimmung der Resonanzfrequenz des Resonators (2).

9. Vorrichtung nach Anspruch 8, wobei die Auswerteinrichtung eine im Halbleitersubstrat (3) angeordnete interne Auswerteeinrichtung (17) ist.

10. Vorrichtung nach Anspruch 8, wobei die Auswerteinrichtung eine außerhalb des Halbleitersubstrats angeordnete externe Auswerteeinrichtung ist.

11. Vorrichtung nach einem der Ansprüche 1 bis 10, wobei der Resonator (2) eine Schutzschicht (12) aufweist und die chemisch sensitive Beschichtung (10) auf der Schutzschicht (12) aufgebracht ist.

12. Vorrichtung nach einem der Ansprüche 1 bis 11, wobei eine Vielzahl von Resonatoren (2) zu einer Resonatormatrix (26) zusammengefasst ist und jeder der Resonatoren (2) ein Matrixelement (27) der Resonatormatrix (26) bildet.

13. Vorrichtung nach Anspruch 12, wobei jeder der Resonatoren (2) der Resonatormatrix (26) der Detektion einer bestimmten Substanz dient.

14. Vorrichtung nach Anspruch 12 oder 13, wobei ein Abstand (28) zwischen benachbarten Matrixelementen (27) aus dem Bereich von einschließlich 50 $\mu$m bis einschließlich 1000 $\mu$m ausgewählt ist.

15. Verfahren zur Detektion mindestens einer Substanz eines Fluids unter Verwendung einer Vorrichtung nach einem der Ansprüche 1 bis 14 mit folgenden Verfahrensschritten:

   a) Zusammenbringen des Fluids und des piezoakustischen Resonators derart, dass die Substanz am Oberflächenabschnitt des Resonators sorbiert werden kann und
   b) Bestimmen einer Resonanzfrequenz des Resonators, wobei aus der Resonanzfrequenz auf die am Oberflächenabschnitt sorbierte Menge der Substanz geschlossen wird.

16. Verfahren nach Anspruch 15, wobei die Resonanzfrequenz in Gegenwart des Fluids bestimmt wird.

17. Verfahren nach Anspruch 15 oder 16, wobei die Resonanzfrequenz in Abwesenheit des Fluids bestimmt wird.

18. Verfahren nach Anspruch 17, wobei als Fluid eine Flüssigkeit verwendet wird und nach dem Zusammenbringen des Fluids und des Resonators und vor dem Bestimmen der Resonanzfrequenz das Fluid derart entfernt wird, dass die Substanz am Oberflächenabschnitt des Resonators sorbiert bleibt.

**Claims**

1. Device (1) for detecting at least one substance of a fluid (9), the device having at least one piezoacoustic resonator (2) comprising

   - at least one piezoelectric layer (4),
   - an electrode (5, 6) disposed on the piezoelectric layer (4)
   - at least one further electrode (6, 5) disposed on the piezoelectric layer (4), and
   - a surface section (8) for sorption of the substance of the fluid (9),
   - wherein the piezoelectric layer (4), the electrodes (5, 6) and the surface section (8) are arranged with respect to one another in such a way that an electrical actuation of the electrodes (5, 6) leads to a thickness oscillation of the resonator (2) at a resonance frequency and the resonance frequency is dependent on an amount of the substance sorbed on the surface section (8),
   - a layer thickness (7) of the piezoelectric layer (4) is chosen from the range of 0.1 $\mu$m inclusive to 20 $\mu$m inclusive and
   - the resonance frequency of the thickness oscillation is chosen from the range of 500 MHz inclusive to 10 GHz inclusive,
   - the surface section (8) for sorption of the substance of the fluid (9) is formed by a chemically sensitive coating (10) of the resonator (2),
   - the resonator (2) is disposed on a semiconductor substrate (3), and
   - at least one device (15) is present to provide acoustic insulation of the resonator (2) and of the semiconductor substrate (3),
   **characterised in that**

- the device serving for acoustic insulation is an acoustic mirror integrated in the substrate.

2. Device according to claim 1, wherein the chemically sensitive coating has molecules for detecting the substance.

3. Device according to claim 1, wherein the chemically sensitive coating has an immobilization layer for connecting the resonator and the molecules for detecting the substance.

4. Device according to one of claims 1 to 3, wherein the resonator (2) has a lateral extension (11) which is chosen from the range of 20 $\mu$m inclusive to 1000 $\mu$m inclusive.

5. Device according to one of claims 1 to 4, wherein the thickness oscillation of the resonator (2) is chosen from the longitudinal oscillation (52) group and/or the thickness shear mode oscillation (51) group.

6. Device according to one of claims 1 to 5, wherein the piezoelectric layer (4) has a piezoelectric material which is chosen from the lead zirconate titanate, zinc oxide and/or aluminium nitride group.

7. Device according to one of claims 1 to 6, wherein the surface section (8) for sorption of the substance of the fluid is disposed at a recess (13) of the semiconductor substrate (3).

8. Device according to one of claims 1 to 7, wherein at least one evaluation device (17, 18) is present for the purpose of determining the resonance frequency of the resonator (2).

9. Device according to claim 8, wherein the evaluation device is an internal evaluation device (17) disposed in the semiconductor substrate (3).

10. Device according to claim 8, wherein the evaluation device is an external evaluation device disposed outside of the semiconductor substrate.

11. Device according to one of claims 1 to 10, wherein the resonator (2) has a protective layer (12) and the chemically sensitive coating (10) is applied to the protective layer (12).

12. Device according to one of claims 1 to 11, wherein a plurality of resonators (2) are combined to form a resonator array (26) and each of the resonators (2) forms an array element (27) of the resonator array (26).

13. Device according to claim 12, wherein each of the resonators (2) in the resonator array (26) serves to detect a specific substance.

14. Device according to claim 12 or 13, wherein a spacing (28) between adjacent array elements (27) is chosen from the range of 50 $\mu$m inclusive to 1000 $\mu$m inclusive.

15. Method for detecting at least one substance of a fluid using a device according to one of claims 1 to 14, the method comprising the following steps:

a) bringing the fluid and the piezoacoustic resonator into contact in such a way that the substance can be sorbed on the surface section of the resonator, and
b) determining a resonance frequency of the resonator, wherein the amount of the substance sorbed on the surface section can be deduced from the resonance frequency.

16. Method according to claim 15, wherein the resonance frequency is determined in the presence of the fluid.

17. Method according to claim 15 or 16, wherein the resonance frequency is determined in the absence of the fluid.

18. Method according to claim 17, wherein a liquid is used as the fluid and after the fluid and the resonator are brought into contact and before the resonance frequency is determined the fluid is removed in such a way that the substance remains sorbed on the surface section of the resonator.

**Revendications**

1.  Dispositif (1) pour détecter au moins une substance d'un fluide (9), comprenant au moins un résonateur piézoacoustique (2) ayant

    - au moins une couche piézoélectrique (4),
    - une électrode (5, 6) disposée sur la couche piézoélectrique (4),
    - au moins une autre électrode (6, 5) disposée sur la couche piézoélectrique (4), et
    - une portion de surface (8) pour la sorption de la substance du fluide (9),
    - la couche piézoélectrique (4), les électrodes (5, 6) et la portion de surface (8) étant disposées les unes contre les autres de telle façon qu'une excitation électrique des électrodes (5, 6) provoque une vibration d'épaisseur du résonateur (2) à une fréquence de résonance et que la fréquence de résonance dépende d'une quantité absorbée de la substance au niveau de la portion de surface,
    - une épaisseur de couche (7) de la couche piézoélectrique (4) étant choisie dans la plage allant de 0,1 $\mu$m inclus à 20 $\mu$m inclus et
    - la fréquence de résonance de la vibration d'épaisseur étant choisie dans la plage allant de 500 MHz inclus à 10 GHz inclus,
    - la portion de surface (8) pour la sorption de la substance du fluide (9) étant formée par un revêtement (10) chimiquement sensible du résonateur (2),
    - le résonateur (2) étant disposé sur un substrat semi-conducteur (3) et
    - au moins un moyen (15) destiné à l'isolation acoustique du résonateur (2) et du substrat semi-conducteur (3) étant prévu,
    **caractérisé en ce que**
    - le moyen d'isolation acoustique est un miroir acoustique intégré au substrat.

2.  Dispositif selon la revendication 1, le revêtement chimiquement sensible comportant des molécules de reconnaissance de la substance.

3.  Dispositif selon la revendication 2, le revêtement chimiquement sensible comportant une couche d'immobilisation destinée à lier le résonateur et les molécules de reconnaissance de la substance.

4.  Dispositif selon l'une des revendications 1 à 3, le résonateur (2) ayant une extension latérale (11) choisie dans la gamme allant de 20 $\mu$m inclus à 1000 $\mu$m inclus.

5.  Dispositif selon l'une des revendications 1 à 4, la vibration d'épaisseur du résonateur (2) étant choisie dans le groupe comprenant la vibration longitudinale (52) et/ou la vibration de cisaillement d'épaisseur (51).

6.  Dispositif selon l'une des revendications 1 à 5, la couche piézoélectrique (4) comportant un matériau piézoélectrique choisi dans le groupe comprenant le zirconate-titanate de plomb, l'oxyde de zinc et/ou le nitrure d'aluminium.

7.  Dispositif selon l'une des revendications 1 à 6, la portion de surface (8) pour la sorption de la substance du fluide étant disposée au niveau d'un évidement (13) du substrat semi-conducteur (3).

8.  Dispositif selon l'une des revendications 1 à 7, au moins un moyen d'évaluation (17, 18) étant prévu pour déterminer la fréquence de résonance du résonateur (2).

9.  Dispositif selon la revendication 8, le moyen d'évaluation étant un moyen d'évaluation interne (17) placé dans le substrat semi-conducteur (3).

10. Dispositif selon la revendication 8, le moyen d'évaluation étant un moyen d'évaluation externe placé hors du substrat semi-conducteur (3).

11. Dispositif selon l'une des revendications 1 à 10, le résonateur (2) comprenant une couche de protection (12) et le revêtement (10) chimiquement sensible étant appliqué sur la couche de protection (12).

12. Dispositif selon l'une des revendications 1 à 11, une pluralité de résonateurs (2) étant réunis en une matrice de résonateurs (26) et chacun des résonateurs (2) formant un élément de matrice (27) de la matrice de résonateurs (26).

**13.** Dispositif selon la revendication 12, chacun des résonateurs (2) de la matrice de résonateurs (26) servant à la détection d'une substance déterminée.

**14.** Dispositif selon la revendication 12 ou 13, un écartement (28) entre éléments de matrice (27) voisins étant choisi dans la plage allant de 50 $\mu$m inclus à 1000 $\mu$m inclus.

**15.** Procédé pour détecter au moins une substance d'un fluide en utilisant un dispositif selon l'une des revendications 1 à 14, comprenant les étapes de procédé suivantes :

a) mise en contact du fluide et du résonateur piézoacoustique de façon à ce que la substance puisse être absorbée au niveau de la portion de surface du résonateur et
b) détermination d'une fréquence de résonance du résonateur, la quantité absorbée de la substance au niveau de la portion de surface étant déduite de la fréquence de résonance.

**16.** Procédé selon la revendication 15, la fréquence de résonance étant déterminée en présence du fluide.

**17.** Procédé selon la revendication 15 ou 16, la fréquence de résonance étant déterminée en l'absence du fluide.

**18.** Procédé selon la revendication 17, comme fluide étant utilisé un liquide, et après la mise en contact du fluide et du résonateur et avant la détermination de la fréquence de résonance, le fluide étant éliminé de façon à ce que la substance reste absorbée au niveau de la portion de surface.

FIG 1A

FIG 1B

FIG 1C

**FIG 1D**

**FIG 1E**

**FIG 1F**

## FIG 2A

## FIG 2B

## FIG 2C

## FIG 3A

## FIG 3B

## FIG 4A

2,27    ←28→    2,27    2,27    26

1    3

## FIG 4B

2,27    5    3

26    29

6    29

1    29

29

## FIG 4C

2,27    5

23

26    6

1    3

## FIG 5A

## FIG 5B

## FIG 6

| Zusammenbringen des Fluids mit der Substanz und des Oberflächenabschnitts zur Sorption der Substanz | 61 |
| --- | --- |
| Bestimmen der Resonanzfrequenz des Resonators | 62 |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5932953 A

- US 5075641 A

### In der Beschreibung aufgeführte Nicht-Patentliteratur

- **I. WILLNER ; E. KATZ.** *Angew. Chem.,* 2000, vol. 112, 1230-1269
- **C. KÖßLINGER et al.** *Biosensors & Bioelectronics,* 1992, vol. 7, 397-404
- **G. SAUERBREY.** *Zeitschrift für Physik,* 1959, vol. 155, 206-222

- **V. FERRARI ET AL.** *Sensors and Actuators,* 2000, vol. 68, 81-87
- **H. BALTES.** *Proceedings of the IEEE,* 1998, vol. 86 (8), 1660-1678